(19) **Europäisches Patentamt**
**European Patent Office**
**Office européen des brevets**

(11) **EP 1 192 897 B1**

(12) **EUROPÄISCHE PATENTSCHRIFT**

(45) Veröffentlichungstag und Bekanntmachung des Hinweises auf die Patenterteilung:
**28.03.2007 Patentblatt 2007/13**

(51) Int Cl.:
*A61B 5/0452* (2006.01)        *A61B 5/0468* (2006.01)
*G06F 17/00* (2006.01)

(21) Anmeldenummer: **01250335.5**

(22) Anmeldetag: **25.09.2001**

(54) **Risikomonitoring**

Risk monitoring

Contrôle de risques

(84) Benannte Vertragsstaaten:
**AT BE CH CY DE DK ES FI FR GB GR IE IT LI LU MC NL PT SE TR**

(30) Priorität: **26.09.2000 DE 10048649**

(43) Veröffentlichungstag der Anmeldung:
**03.04.2002 Patentblatt 2002/14**

(73) Patentinhaber: **BIOTRONIK GmbH & Co. KG**
**12359 Berlin (DE)**

(72) Erfinder:
• **Hutten, Helmut**
**8042 Graz (AT)**

• **Hribernigg, Martin**
**8010 Graz (AT)**
• **Rauchegger, Günter**
**8051 Graz-Gosting (AT)**

(74) Vertreter: **Lindner-Vogt, Karin L.**
**BIOTRONIK GmbH & Co. KG**
**Corporate Intellectual Properties**
**Woermannkehre 1**
**12359 Berlin (DE)**

(56) Entgegenhaltungen:
WO-A-99/23944        DE-A- 4 405 827
US-A- 5 437 285        US-A- 5 471 991

EP 1 192 897 B1

## Beschreibung

Die Erfindung betrifft eine Risikobewertungsvorrichtung insbesondere für das Risiko einer schweren Herzfunktionsstörung wie des plötzlichen Herztodes, mit einer Eingangseinheit, die ausgebildet ist, ein Elektrokardiogramm als Eingangssignal aufzunehmen oder einzulesen und ein entsprechendes, mehrere Herzzyklen und entsprechende Intervalle umfassendes Ausgangssignal auszugeben, und mit einer mit der Eingangseinheit verbundenen Detektionseinheit, welche ausgebildet ist, Ereignisse vorgegebener Art in dem Ausgangssignal zu detektieren.

Die Intervalle können R-R Intervalle sein, aber auch p-p Intervalle oder andere Intervalle eines Elektrokardiogramms. Üblicherweise enthält ein Elektrokardiogramm eine Vielzahl von Intervallen normaler Länge. Insbesondere im Fall von Extrasystolen kann es jedoch zu verkürzten oder verlängerten Intervallen kommen, die nicht einem Normalintervall entsprechen.

Aus der WO 99/23944 sind ein Verfahren und eine Vorrichtung bekannt, die dem Auswerten von Elektrokardiogrammen dienen, mit dem Ziel, durch Auswertung von Elektrokardiogrammen im Bereich von Extrasystolen Parameter zu gewinnen, die dem individuellen Risiko einer Person zugeordnet werden können.

Im Zeitbereich wird dazu beispielsweise ein "Onset" ermittelt. Dies ist die Differenz der Mittelwerte der letzten Normalen RR-Intervalle vor der Extrasystole und der ersten normalen RR-Intervalle nach der Extrasystole.

Ein anderer Parameter im Zeitbereich ist der "Slope". Dies ist die größte Frequenzverlangsamung innerhalb einer Sequenz von mehreren Herzschlagintervallen nach einer Extrasystole über eine vorgegebene Anzahl aufeinanderfolgender RR-Intervalle. Ermittelt wird die Steigung der die Frequenzverlangsamung beschreibenden Regressionsgraden. Im Zusammenhang mit dem Slope wird dessen Korrelationskoeffizient bestimmt, also ein Maß für die Regelmäßigkeit des Slope, welches durch numerische Mittelwertbildung mehrerer aufeinanderfolgender Slope-Werte gebildet wird.

Aus der WO 99/23944 ist es bekannt, dass bei geringem Onset, flachem Slope oder niedrigem Korrelationskoeffizienten des Slope das Risiko, im weiteren Verlauf zu versterben, signifikant erhöht ist.

In DE 44 05 827 ist ein Verfahren mit einer Einrichtung zum Verarbeiten eines Langzeit-EKG's beschrieben. Dabei werden als Ausnahmeintervalle bezeichnete Intervalle addiert und die aufsummierte Intervalllänge ausgewertet. Ausnahmeintervalle sind insbesondere solche Intervalle, die eine kürzere Dauer als eine Normlänge haben.

Der Erfindung liegt die Aufgabe zugrunde, eine zur Risikobewertung geeignete Vorrichtung anzugeben, die das Risiko schwerer Herzfunktionsstörungen mit möglichst hoher Sensitivität und mit möglichst hoher Spezifität bestimmen kann. Hohe Sensitivität bedeutet, dass möglichst viele der Kandidaten für einen plötzlichen Herztod gewarnt werden und nur wenige Kandidaten übersehen werden, das heißt ungewarnt bleiben, wobei allerdings auch Personen, bei denen sich das Risiko im weiteren Verlauf nicht bestätigt, gewarnt werden. Hohe Spezifität bedeutet, dass eine Warnung bei den meisten Patienten, die nicht gefährdet sind, unterbleibt, wobei in Wirklichkeit jedoch auch einige nicht gefährdete Patienten irrtümlich gewarnt (d. h. beunruhigt) werden.

Erfindungsgemäß wird diese Aufgabe durch eine Vorrichtung der eingangs genannten Art gelöst, die eine Summationseinheit umfasst, welche mit der Detektionseinheit und der Eingangseinheit verbunden und ausgebildet ist, die Länge vorbestimmter, mit einem detektierten Ereignis in Verbindung stehender Intervalle innerhalb des Eingangssignals aufzuaddieren und einen entsprechenden Summenparameter auszugeben, sowie eine Auswerteeinheit, welche zur Auswertung des Summenparameters unter vorgegebenen Bedingungen und zur Ausgabe eines von der Auswertung abhängigen Risikoparameters ausgebildet ist. Die Auswertung des Summenparameters in der Auswerteeinheit zur Bildung des Risikoparameters erfolgt vorzugsweise dahingehend, dass der zeitliche Verlauf des Summenparamters analysiert wird und ein dem jeweiligen zeitlichen Trendparameter entsprechender Risikoparameter gebildet wird.

Die Detektionseinheit ist ausgebildet, ventrikuläre Extrasystolen als vorgegebenes Ereignis zu detektieren.

Die Detektionseinheit ist mit einer Selektionseinheit verbunden, die ausgebildet ist unter den detektierten Ereignisse solche Ereignisse zu selektieren, die eine vorgegebene Eigenschaft aufweisen.

Die Selektionseinheit ist dabei vorzugsweise ausgebildet, solche ventrikulären Extrasystolen zu selektieren, die mit einer Änderung der Morphologie eines der Extrasystole zugeordneten QRS-Komplexes, verglichen mit QRS-Komplexen, die nicht mit einer Extrasystole verknüpft sind, in dem Ausgangssignal der Eingangseinheit einhergehen.

Vorzugsweise ist die Selektionseinheit zusätzlich ausgebildet, solche ventrikulären Extrasystolen zu selektieren, bei denen ein QRS-Komplex verglichen mit den QRS-Komplexen vorangegangener Intervalle mindestens 20% verfrüht ist. Besonders bevorzugt ist eine Selektionseinheit, die zusätzlich ausgebildet ist, solche ventrikulären Extrasystolen zu selektieren, auf die ein gegenüber vorangegangenen Intervallen um mindestens 10% verlängertes Intervall folgt. Zur noch weitergehenden Selektion ist die Selektionseinheit ausgebildet, eine vorgegebene Anzahl von keine Extrasystole enthaltenden Intervallen vor und nach einer ventrikulären Extrasystole für die Bildung des Summenparameters zu selektieren. Diese Maßnahmen tragen jeweils für sich und insbesondere zusammengenommen dazu bei, dass Fehlklassifikationen durch Störungen im Signal wie Rauschen, Brummen oder andere dem Elektrodardiogramm überlagerte Signale weitgehend vermieden werden. Die Anzahl der jeweils berücksichtigten einer Extrasystole vorangehenden oder nachfolgenden Intervalle kann beispielsweise zwischen 2 und 20 betragen und dazu verwendet werden, entsprechend weiteren Bedingungen die günstigsten Werte für Sensitivität und Spezifität vorzugeben. Die Reihenfolge der genannten Maßnahmen kann dabei den Gegebenheiten des Signals in Hinblick auf die Signalqualität und Verarbeitungszeit angepasst und die Anordnung der Maßnahmen vertauscht werden.

In einer weiteren bevorzugten Ausführungsvariante umfasst die Detektionseinheit eine Intervallselektionseinheit, die ausgebildet ist, solche Intervalle für die Bildung des Summenparameters zu selektieren, denen mindestens zwei ventrikuläre Extrasysolen zugeordnet sind, welche innerhalb eines vorgegebenen Zeitraums auftreten. Dieser Zeitraum hat vorzugsweise die Größenordnung einer Stunde.

Ergänzend besteht die Lösung der Aufgabe in einer Risikobewertungsvorrichtung mit einer Eingangseinheit, die ausgebildet ist, ein elektrokardiographisches Eingangssignal aufzunehmen oder einzulesen und ein entsprechendes, mehrere Herzzyklen und entsprechende Intervalle umfassendes Ausgangssignals auszugeben und mit einer ersten Auswerteeinheit, welche zumindest mittelbar mit der Eingangseinheit verbunden ist und zur Auswertung des Ausgangssignals unter vorgegebenen Bedingungen und zur Ausgabe eines von der Auswertung abhängigen Risikoparameters ausgebildet ist, gekennzeichnet durch eine zweite Auswerteeinheit, die mit der ersten Auswerteeinheit zur Übernahme des Risikoparameters als einem ersten Parameter verbunden ist und zumindest einen Eingang für zumindest einen weiteren Parameter aufweist sowie ausgebildet ist, ein einer Wahrscheinlichkeit einer schweren Herzfunktionsstörung entsprechendes Risokosignal durch Linearkombination der Parameter, wie sie vorzugsweise durch die logistische Regression bestimmt wird, oder durch nichtlineare Kombination der Parameter zu errechnen.

Es hat sich gezeigt, dass nicht nur der wie zuvor beschriebene, aus dem Summenparameter gebildete Risikoparameter ein geeigneter Eingangswert für die logistische Regressionsanalyse ist, sondern beispielsweise auch der eingangs beschriebene Onset oder der Slope sowie die statistische Schwankungsbreite dieser Werte. Jedoch ergeben sich besonders spezifisch und signifikante Ergebnisse, wenn der Summenparameter oder dessen zeitlicher Verlauf neben Alter und Blutdruck eines Patienten in die Linearkombination der Parameter eingehen.

Zusammengefasst bestehen die wesentlichen Aspekte der Erfindung zum einen in der Bildung des beschriebenen Summenparameters und zum anderen in der Verknüpfung verschiedener Risikoparameter durch eine Linearkombination. Beide Aspekte tragen alleine und unabhängig voneinander zu einer Verbesserung der Sensitivität und der Spezifität bei. Besonders gut wird die Aufgabe durch eine Risikobewertungsvorrichtung gelöst, bei der der Summenparameter einer der Risikoparameter ist, die in die Parameterkombination eingehen.

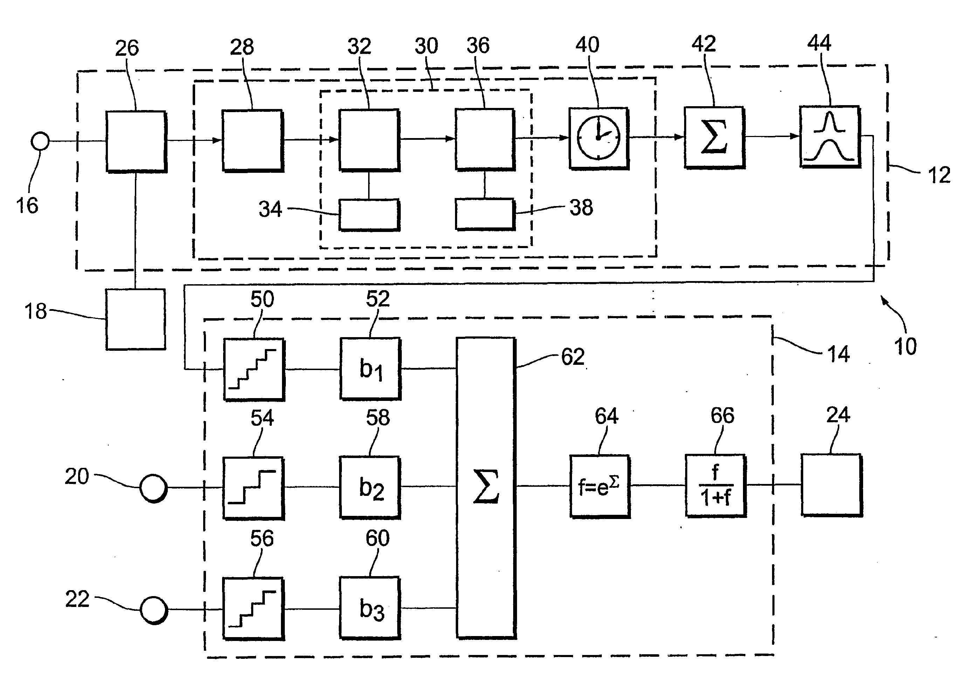

Die Erfindung soll nun anhand eines Ausführungsbeispiels unter Bezug auf die Figur näher erläutert werden. Die Figur zeigt ein schematisches Blockschaltbild einer erfindungsgemäßen Risikobewertungsvorrichtung.

Die Risikobewertungsvorrichtung 10 umfasst als Hauptbestandteil einen EKG-Risikoparameterzweig 12 und einen Risikoanalysator 14, die im Ausführungsbeispiel in vorteilhafter Weise miteinander verbunden sind, aber auch separat voneinander realisiert sein können.

Eingangsseitig weist der EKG-Risikoparameterzweig 12 einen Anschluss für einen EKG-Messwertaufnehmer 16 auf. Über einen zweiten Anschluss kann alternativ auch ein auf einem Datenträger 18 gespeichertes EKG eingelesen werden. Ausgangsseitig ist der EKG-Risikoparameterzweig 12 mit einem Eingang des Risikoanalysators 14 verbunden. Der Risikoanalysator 14 weist im Ausführungsbeispiel zwei weitere Parametereingänge 20 und 22 auf.

Ausgangsseitig ist der Risikoanalysator 14 mit einer Ausgabeeinheit 24 verbunden. Die Ausgabeeinheit kann eine Schnittstelle für weitere nachgeschaltete Einheiten aufweisen, kann eine Anzeige umfassen, um einen berechneten Wert für beispielsweise das Risiko eines plötzlichen Herztodes anzuzeigen oder aber auch eine Schwellwerteinheit umfassen, die immer dann, wenn das berechnete Risiko einen Grenzwert überschreitet, ein Alarmsignal ausgibt. Eine solche Schwellwerteinheit kann darüber hinaus mit einer Therapieeinheit verbunden sein, die bei Überschreiten des Schwellwertes ausgelöst und beispielsweise zu einer medikamentösen Therapie oder einer Elektrotherapie des Herzens veranlasst wird.

In einer Variante ist die abgebildete Risikobewertungsvorrichtung 10 so ausgeführt, dass sie Teil eines implantierten Gerätes ist, welches mittels des EKG-Eingangs 16 und einer mit diesem verbundenen Eingangseinheit 26 in der Lage ist, als Eingangssignal ein direkt vom Herzen abgeleitetes oder von einem anderen Ort im Körperinneren gewonnenes EKG aufzunehmen. An die EKG-Einheit 16 kann ein Messwertaufnehmer beispielsweise eine Sense-Elektrode einer Elektrodenleitung angeschlossen werden, wie sie vorzugsweise bei Herzschrittmachern eingesetzt wird. Die Eingangseinheit 26 umfasst die nötigen Eingangsverstärker und Filter, um das Eingangssignal aufzubereiten und an ihrem Ausgang ein aufbereitetes EKG-Signal zur Verfügung zu stellen.

Das aufbereitete EKG-Signal wird einer Detektionseinheit 28 zugeführt, die ausgebildet ist, die im Eingangssignal enthaltenen QRS-Komplexe und insbesondere verschiedene Arten von Extrasystolen in dem aufbereiteten EKG-Signal zu detektieren. Solche Extrasystolen sind unter anderem supraventrikuläre Extrasystolen, ventrikuläre Extrasystolen ohne Änderung der Morphologie des entsprechenden QRS-Komplexes in dem EKG (VES-1) und ventrikuläre Extrasystolen, die mit einer Änderung der Morphologie des entsprechenden QRS-Komplexes einhergehen (VES-2), nachfolgend werden im Zusammenhang mit Extrasystolen nur dann nähere Angaben nach dem hier beschriebenen Klassifikationsschema gemacht, wenn dieses zur Erklärung der Funktion und des Anspruches erforderlich ist.

Von der Detektionseinheit 28 gelangt das aufbereitete EKG-Signal zusammen mit der Information über detektierte Extrasystolen zu einer Selektionseinheit 30, welche außer üblichen Speicherelementen eine Morphologievergleichseinheit 32 umfasst, die dazu ausgebildet ist, Veränderungen der Morphologie eines QRS-Komplexes zu detektieren. Dazu ist die Morphologievergleichseinheit 32 mit einem Signalabschnittsspeicher 34 verbunden, in dem jeweils die Form oder daraus extrahierte Parameter desjenigen QRS-Komplexes gespeichert sind, der für das zuletzt erfasste Bezugsintervall ermittelt worden ist. Das Bezugsintervall ist dabei ein Intervall, das einer vorgegebenen Dauer oder einer vorgegebenen Anzahl von Normalereignissen entspricht und keine Extrasystole enthält.

Auf Grund des Vergleiches des in dem Signalabschnittspeicher 34 gespeicherten Signalabschnittes oder der extrahierten Parameter mit dem entsprechenden, einer ventrikulären Extrasystole zugeordneten Signalabschnitt oder dessen extrahierten Parametern ist die Morphologievergleichseinheit 32 in der Lage, Veränderungen der Morphologie eines QRS-Komplexes im Zusammenhang mit einer ventrikulären Extrasystole zu detektieren und ein entsprechendes Markersignal für die ventrikuläre Extrasystole mit QRS-Komplex-Morphologieveränderung (Typ VES-2) und das der ventrikulären Extrasystole mit QRS-Morphologieveränderung (Typ VES-2) vorausgehende R-R Intervall und das nachfolgende R-R Intervall auszugeben. Im Anschluss an die Morphologievergleichseinheit 32 wird das aufbereitete EKG-Signal mit der Information über aufgetretene VES-2 Extrasystolen einer Referenzintervallvergleichseinheit 36 der Selektionseinheit 30 zugeführt. Die Referenzintervallvergleichseinheit 36 dient der weiteren Selektion der eine ventrikuläre Extrasystole enthaltenden Intervalle. Die Referenzintervallvergleichseinheit 36 ist dazu mit einem Referenzintervallspeicher 38 verbunden in dem ein aus dem Durchschnitt der letzten Normalintervalle abgeleitetes Referenzintervall gespeichert ist. Bevorzugt handelt es sich dabei um eine relativ geringe Zahl von Normalintervallen, beispielsweise um fünf Normalintervalle, um eine hinreichend rasche Anpassung an physiologische Veränderungen, wie sie ständig und ohne Zusammenhang mit Extrasystolen auftreten, zu ermöglichen. Die Referenzintervallvergleichseinheit 36 ist so ausgebildet, dass sie nur solche, eine VES-2 Extrasystole enthaltende Intervalle selektiert, in denen die Extrasystole gegenüber dem Referenzintervall mindestens um einen vorgegebenen Grenzwert (bevorzugt 20 %) verfrüht ist und die eine postextrasystolische Pause aufweisen, die mindestens um einen Grenzwert (bevorzugt 10 %) länger ist, als die Pause nach der Systole in dem Referenzintervall. Weiterhin ist die Referenzintervallvergleichseinheit 36 ausgebildet, nur solche VES-2 Extrasystolen enthaltende Intervalle zu selektieren, denen eine vorgegebene Zahl von Normalintervallen vorangeht und die von einer ebenfalls vorgegebenen Zahl Normalintervallen gefolgt werden.

Die Morphologievergleichseinheit 32 in Verbindung mit dem Signalabschnittspeicher 34 kann dabei nach den Gegebenheiten des Signals in Hinblick auf die Signalqualität und Verarbeitungszeit mit der Referenzintervallvergleichseinheit 36 in Verbindung mit dem Referenzintervallspeicher 38 vertauscht werden.

Am Ausgang der Referenzintervallvergleichseinheit 36 bzw. der Morphologievergleichseinheit 32, im Fall der oben beschriebenen Vertauschung der Funktionseinheiten, steht die Information über das aufbereitete EKG-Signal und die selektierten Intervalle zur Verfügung und liegt am Eingang einer Intervallselektionseinheit 40 an. Die Intervallselektionseinheit 40 führt eine weitere Selektion derart durch, dass nur solche Intervalle selektiert werden, die sich auf mindestens zwei VES-2 Extrasystolen beziehen, welche innerhalb einer vorgegebenen Zeit, konkret zum Beispiel innerhalb einer Stunde, aufeinander folgen. Falls somit innerhalb dieser Zeit nur eine VES-2 Extrasystole auftritt, wird das entsprechende Intervall nicht selektiert. Falls mehr als eine VES-2 Extrasystole innerhalb einer Stunde auftritt, werden die entsprechenden Intervalle selektiert. Am Ausgang der Intervallselektionseinheit 40 und somit am Eingang der Summationseinheit 42 steht wiederum das aufbereitete EKG-Signal sowie die Information über die ausgewählten Intervalle zur Verfügung. Die Summationseinheit 42 ist ausgebildet, die Summe der Zeitdauer der ausgewählten Intervalle zu bilden. Weiterhin ist die Summationseinheit 42 ausgebildet, die Summe der ausgewählten Intervalle für jeweils eine vorgegebene Zeit, vorzugsweise 24 Stunden zu bestimmen, wahlweise jeweils bei Null beginnend oder gleitend über den vorgegebenen, der letzten VES-2 Extrasystole vorangegangenen Zeitraum. Es entsteht somit eine Art Dichtefunktion über das Auftreten von Intervallen, die von der Intervallselektion 40 detektiert werden. Diese liegt am Eingang einer ersten Auswerteeinheit 44 an. Die erste Auswerteeinheit 44 ist ausgebildet, den Trendverlauf der eingehenden Summenparameter zu bestimmen.

Der Summenparameter selbst ist bereits ein Indikator für das Risiko eines plötzlichen Herztodes. Ist der Wert des Summenparamters groß, ist das Risiko, an einem plötzlichen Herztod zu versterben, größer als bei einem kleinen Wert des Summenparameters. Weiterhin deutet ansteigender Trendverlauf, unter Berücksichtigung der Streuung des Summenparameters, auf eine Erhöhung des Risikos hin, an einem plötzlichen Herztod zu versterben.

Der am Ausgang des EKG-Risikoparameterzweiges 12 anliegende Summenparameter oder dessen Trendverlauf kann beispielsweise einer Schwellwerteinheit zugefügt werden, die immer dann, wenn der Summenparameter oder dessen Trendverlauf einen bestimmten Grenzwert überschreitet, einen Alarm gibt, der auf ein hohes Risiko für einen plötzlichen Herztod hindeutet. Mit der Schwellwerteinheit kann auch eine Einheit zur Abgabe eines Medikamentes oder zur Durchführung einer sonstigen präventiv-therapeutischen Maßnahme verbunden sein.

In dem Ausführungsbeispiel liegt der Summenparameter oder dessen Trendverlauf an einem von drei Eingängen des Risikoanalysators 14 an. Er wird zunächst einer Intervallskalierungseinheit 50 des Risikoanalysators 14 zugeführt, in der der kontinuierliche Summenparameter oder dessen Trendverlauf intervallskaliert wird. Der das jeweilige Intervall, in das ein aktueller Summenparameter oder dessen Trendverlauf fällt, beschreibende Wert wird in einer Multiplikationseinheit 52 mit einem zuvor bestimmten Koeffizienten multipliziert. Im Ausführungsbeispiel ist dies der Koeffizient $b_1$. Die Muliplikationseinheit 52 ist dazu mit der Intervallskalierungseinheit 50 verbunden.

Auch die an den Parametereingängen 20 und 22 anliegenden Signale werden je nach ihrer Größe in entsprechenden Intervallskalierungseinheiten 54 und 56 intervallskaliert und der das jeweilige Intervall beschreibende Skalenwert in Multiplikationseinheiten 58 und 60 mit den zuvor bestimmten Koeffizienten $b_2$ und $b_3$ multipliziert. An den Ausgängen der Multiplikationseinheiten 52, 58 und 60 stehen somit drei unterschiedliche, mit den jeweiligen Koeffizienten multiplizierte Skalenwerte zur Verfügung. Diese drei Werte werden, zusammen mit einer Konstanten 61, in einer Summeneinheit 62 miteinander addiert. Am Ausgang der Summeneinheit 62 steht eine Parametersumme zur Verfügung, die in einer darauffolgenden Exponentialeinheit 64 derart verrechnet wird, dass am Ausgang der Exponentialeinheit 64 ein Wert anliegt, der der Eulerzahl $e^{Parametersumme}$ entspricht. Dieser Wert wird in der darauffolgenden Recheneinheit 66 durch die Summe aus diesem Wert plus 1 geteilt. Am Ausgang der Recheneinheit 66 steht ein Risikofaktor zur Verfügung, der die Wahrscheinlichkeit, an einem plötzlichen Herztod zu sterben, beschreibt und in dessen Bestimmung nicht nur der Summenparameter aus dem EKG-Risikoparameterzweig 12 eingeht, sondern über die Parametereingänge 20 und 22 auch solche Risikofaktoren wie das Lebensalter oder der Blutdruck. Diese Risikoparameter werden in dem Risikoanalysator 14 nach dem Ansatz der logistischen Regression miteinander verrechnet. Der beschriebene Aufbau des Risikoanalysators 14 führt dabei zu einer Berechnung, wobei der dessen Ausgangswert p von den Eingangswerten X1, X2 und X3 wie folgt abhängt:

$$p = \frac{e^{(b_1 \cdot X1 + b_2 \cdot X2 + b_3 \cdot X3 + a)}}{1 + e^{(b_1 \cdot X1 + b_2 \cdot X2 + b_3 \cdot X3 + a)}}$$

Die Koeffizienten $b_1$, $b_2$ und $b_3$ und a werden dabei auf die für logistische Regressionen übliche Weise abgestimmt.

Es hat sich gezeigt, dass eine entsprechende Risikobewertungsvorrichtung das Risiko eines plötzlichen Herztodes sowohl mit hoher Sensitivität als auch mit hoher Spezifität bestimmen kann. Die Zielsetzung von möglichst großen Werten sowohl für Sensitivität als auch für Spezifität wird bei der erfindungsgemäßen Vorrichtung durch eine der folgenden Maßnahmen allein oder ihre geeignete Kombination erreicht:

- Selektion von VES-2 Extrasystolen nach vorgegebenen Kriterien und Grenzwerten
- Bildung von Summenparametern und daraus abgeleiteten Trendparameters
- Berücksichtigung von Risikoparametern, die anhand eines logistischen Regressionsmodells validiert worden sind
- Bildung von Risikofaktoren, die anhand von Gewichtungskoeffizienten, die entsprechend der Zielvorgabe festgelegt werden, und intervallskalierten Risikoparamtern errechnet werden.

**Patentansprüche**

1. Risikobewertungsvorrichtung insbesondere für das Risiko einer schweren Herzfunktionsstörung wie des plötzlichen Herztodes, mit

   - einer Eingangseinheit (26), die ausgebildet ist, ein elektrokardiographisches Eingangssignal aufzunehmen oder einzulesen und ein entsprechendes, aufbereitetes Ausgangssignal auszugeben, und mit
   - einer mit der Eingangseinheit verbundenen Detektionseinheit (28), welche ausgebildet ist, Ereignisse vorgegebener Art in dem Ausgangssignal zu detektieren,
   - einer Summationseinheit (42), welche mit der Detektionseinheit (28) und der Eingangseinheit (26) verbunden und ausgebildet ist, die Länge vorbestimmter, mit einem detektierten Ereignis in Verbindung stehender Intervalle innerhalb des Eingangssignals aufzuaddieren und einen entsprechenden Summenparameter auszugeben,
   - einer Auswerteeinheit (44), welche zur Auswertung des Summenparameters unter vorgegebenen Bedingungen und zur Ausgabe eines von der Auswertung abhängigen Risikoparameters ausgebildet ist, und

- einer Selektionseinheit (30), die mit der Detektionseinheit (28) verbunden und ausgebildet ist, unter den detektierten Ereignissen solche Ereignisse zu selektieren, die eine oder mehrere vorgegebene Eigenschaften aufweisen,

**dadurch gekennzeichnet, dass**

- die Selektionseinheit (30) ausgebildet ist, solche ventrikulären Extrasystolen zu selektieren, die mit einer Änderung der Morphologie des der Extrasystole zugeordneten QRS-Komplexes gegenüber nicht mit einer Extrasystole verknüpften QRS-Komplexen in dem Ausgangssignal der Eingangseinheit einhergehen, sogenannte VES-2 Extrasystolen.

2. Risikobewertungsvorrichtung nach Anspruch 1, **dadurch gekennzeichnet, dass** die Selektionseinheit (30) ausgebildet ist, solche ventrikulären VES-2 Extrasystolen zu selektieren, bei denen der QRS-Komplex verglichen mit den QRS-Komplexen vorangegangener keine Extrasystolen enthaltender Intervalle mindestens um einen vorgegebenen Grenzwert verfrüht ist.

3. Risikobewertungsvorrichtung nach einem der Ansprüche 1 oder 2, **dadurch gekennzeichnet, dass** die Selektionseinheit (30) ausgebildet ist, solche ventrikulären VES-2 Extrasystolen zu selektieren, auf die ein gegenüber vorangegangenen und keine Extrasystolen enthaltenden Intervalle mindestens um einen vorgegebenen Grenzwert verlängertes Intervall folgt.

4. Risikobewertungsvorrichtung nach einem der Ansprüche 1 bis 3, **dadurch gekennzeichnet, dass** die Selektionseinheit (30) ausgebildet ist, eine vorgegebene Anzahl von von keine Extrasystole enthaltenden Intervallen vor und nach einer VES-2 Extrasystole für die Bildung des Referenzintervalls zu selektieren.

5. Risikobewertungsvorrichtung nach einem der Ansprüche 1 bis 4, **gekennzeichnet durch** eine Intervallselektionseinheit (40), die mit der Selektionseinheit (30) verbunden und ausgebildet ist, solche Intervalle für die Bildung des Summenparameters zu selektieren, die mindestens zwei VES-2 Extrasystolen zugeordnet sind, welche innerhalb eines vorgegebenen Zeitraums auftreten.

6. Risikobewertungsvorrichtung nach Anspruch 5, **dadurch gekennzeichnet, dass** der vorgegebene Zeitraum die Größenordnung von einer Stunde hat.

7. Risikobewertungsvorrichtung nach einem der Ansprüche 1 bis 6, **dadurch gekennzeichnet, dass** die Auswerteeinheit (44) ausgebildet ist, den Trendverlauf durch Auswertung des Summenparameters derart zu bilden, dass der Risikoparameter dem Trend des Summenparameters entspricht.

8. Risikobewertungsvorrichtung nach einem der Ansprüche 1 bis 7, mit einer Eingangseinheit (26), die ausgebildet ist, ein elektrokardiographisches Eingangssignal aufzunehmen oder einzulesen und ein entsprechend aufbereitetes Ausgangssignal auszugeben und mit einer ersten Auswerteeinheit (28, 30, 40, 42, 44), welche zumindest mittelbar mit der Eingangseinheit verbunden ist und zur Auswertung des Ausgangssignals unter vorgegebenen Bedingungen und zur Ausgabe eines von der Auswertung abhängigen Risikoparameters ausgebildet ist, **gekennzeichnet durch** eine zweite Auswerteeinheit (14), die mit der ersten Auswerteeinheit zur Übernahme des Risikoparameters als einem ersten Parameter verbunden ist und zumindest einen Eingang für zumindest einen weiteren Parameter aufweist und ausgebildet ist, ein einer Wahrscheinlichkeit einer schweren Herzfunktionsstörung entsprechendes Risikosignal auf der Basis einer Linearkombination der Parameter, wie sie vorzugsweise **durch** die logistische Regression bestimmt wird, oder **durch** nichtlineare Kombination der Parameter, zu errechnen.

## Claims

1. A risk evaluation device, in particular for the risk of a severe cardiac function disturbance such as sudden cardiac death, having

- an input unit (26) which is implemented to record or input an electrocardiographic input signal and to output a corresponding, conditioned output signal, and having
- a detection unit (28), connected to the input unit, which is implemented to detect events of a predefined type in the output signal,
- a summation unit (42), which is connected to the detection unit (28) and the input unit (26) and is implemented to add up the length of predetermined intervals, connected to a detected event, within the input signal and output a corresponding summation parameter,
- an analysis unit (44), which is implemented to analyze the summation parameter under predefined conditions and to output a risk parameter as a function of the analysis, and
- a selection unit (30), which is connected to the detection unit (28) and is implemented to select those events among the detected events which have one or more predefined properties,

**characterized in that**

- the selection unit (30) is implemented to select those ventricular extrasystoles which accompany a change of the morphology of the QRS complex assigned to the extrasystoles in relation to QRS complexes which are not linked to an extrasystole in the output signal of the input unit, i.e., VES-2 extrasystoles.

2. The risk evaluation device according to Claim 1, **characterized in that** the selection unit (30) is implemented to select those ventricular VES-2 extrasystoles in which the QRS complex, compared to the QRS complexes of preceding intervals not containing extrasystoles, is earlier by at least a predefined limiting value.

3. The risk evaluation device according to one of Claims 1 or 2, **characterized in that** the selection unit (30) is implemented to select those ventricular VES-2 extrasystoles which follow an interval lengthened by at least a predefined limiting value in relation to preceding intervals not containing extrasystoles.

4. The risk evaluation device according to one of Claims 1 through 3, **characterized in that** the selection unit (30) is implemented to select a predefined number of intervals not containing extrasystoles before and after a VES-2 extrasystole for the calculation of the reference interval.

5. The risk evaluation device according to one of Claims 1 through 4, **characterized by** an interval selection unit (40), which is connected to the selection unit (30) and is implemented to select those intervals for the calculation of the summation parameter which are assigned to at least two VES-2 extrasystoles, which occur within a predefined period of time.

6. The risk evaluation device according to Claim 5, **characterized in that** the predefined period of time has an order of magnitude of an hour.

7. The risk evaluation device according to one of Claims 1 through 6, **characterized in that** the analysis unit (44) is implemented to calculate the trend curve by analyzing the summation parameter in such way that the risk parameter corresponds to the trend of the summation parameter.

8. The risk evaluation device according to one of Claims 1 through 7, having an input unit (26), which is implemented to record or input an electrocardiographic input signal and to output a corresponding conditioned output signal, and having a first analysis unit (28, 30, 40, 42, 44), which is at least indirectly connected to the input unit and is implemented to analyze the output signal under predefined conditions and to output a risk parameter as a function of the analysis, **characterized by** a second analysis unit (14) which is connected to the first analysis unit to adopt the risk parameter as a first parameter and has at least one input for at least one further parameter and is implemented to calculate a risk signal corresponding to a probability of a severe cardiac function disturbance on the basis of a linear combination of the parameters, as is preferably determined by logistical regression, or by nonlinear combination of the parameters.

**Revendications**

1. Dispositif d'évaluation de risque en particulier pour le risque d'un grave dysfonctionnement cardiaque comme une brusque mort par arrêt cardiaque, comprenant

- une unité d'entrée (26) qui est réalisée pour enregistrer ou entrer un signal d'entrée d'électrocardiogramme et sortir un signal de sortie approprié et traité, avec
- une unité de détection (28) reliée à l'unité d'entrée, qui est conçue pour détecter des événements du type prédéfini dans le signal de sortie,
- une unité de totalisation (42), qui est reliée à l'unité de détection (28) et à l'unité d'entrée (26), qui est conçue pour additionner la longueur d'intervalles prédéfinis et en liaison avec un événement détecté à l'intérieur du signal d'entrée et sortir un paramètre global correspondant,
- une unité d'analyse (44), qui est conçue pour l'analyse du paramètre global dans des conditions prédéfinies et pour la sortie d'un paramètre de risque dépendant de l'analyse, et
- une unité de sélection (30), qui est reliée à l'unité de détection (28) et est conçue pour sélectionner parmi les événements détectés ceux qui présentent une ou plusieurs propriétés prédéfinies,

**caractérisé en ce que**

- l'unité de sélection (30) est conçue pour sélectionner des extrasystoles ventriculaires qui s'accompagnent d'une modification de la morphologie du complexe QRS attribué à l'extrasystole par rapport à des complexes QRS non associés à une extrasystole dans le signal de sortie de l'unité d'entrée, appelées extrasystoles VES-2.

2. Unité d'évaluation de risque selon la revendication 1, **caractérisé en ce que** l'unité de sélection (30) est conçue pour sélectionner des extrasystoles VES-2 ventriculaires avec lesquelles le complexe QRS est prématuré d'au moins une valeur limite prédéfinie comparé aux complexes QRS d'intervalles précédents et ne contenant pas d'extrasystoles.

3. Dispositif d'évaluation de risque selon l'une quelconque des revendications 1 ou 2, **caractérisé en ce que** l'unité de sélection (30) est conçue pour sélectionner des extrasystoles VES-2 ventriculaires qui sont suivies d'un intervalle prolongé d'au moins une valeur limite prédéfinie par rapport à des intervalles précédents et ne contenant pas d'extrasystoles.

4. Dispositif d'évaluation de risque selon l'une quelconque des revendications 1 à 3, **caractérisé en ce que** l'unité de sélection (30) est conçue pour sélectionner un nombre prédéfini d'intervalles ne contenant pas d'extrasystoles avant et après une extrasystole VES-2 pour la formation de l'intervalle de référence.

5. Dispositif d'évaluation de risque selon l'une quelconque des revendications 1 à 4, **caractérisé par** une unité de sélection d'intervalle (40) qui est reliée à l'unité de sélection (30) et est conçue pour sélectionner des intervalles pour la formation du paramètre global, qui sont attribués à au moins deux extrasystoles VES-2, lesquelles apparaissent à l'intérieur d'une période de temps prédéfinie.

6. Dispositif d'évaluation de risque selon la revendication 5, **caractérisé en ce que** la période prédéfinie est de l'ordre d'une heure.

7. Dispositif d'évaluation de risque selon l'une quelconque des revendications 1 à 6, **caractérisé en ce que** l'unité d'analyse (44) est conçue pour former la courbe de tendance par l'analyse du paramètre global de telle sorte que le paramètre de risque corresponde à la tendance du paramètre global.

8. Dispositif d'évaluation de risque selon l'une quelconque des revendications 1 à 7, avec une unité d'entrée (26) qui est conçue pour enregistrer ou entrer un signal d'entrée d'électrocardiogramme et sortir un signal de sortie préparé en conséquence et avec une première unité d'analyse (28, 30, 40, 42, 44), qui est reliée au moins indirectement à l'unité d'entrée et est conçue pour l'analyse du signal de sortie dans des conditions prédéfinies et pour la sortie d'un paramètre de risque dépendant de l'analyse, **caractérisé par** une seconde unité d'analyse (14), qui est reliée à la première unité d'analyse pour la prise en charge du paramètre de risque en tant que premier paramètre et présente au moins une entrée pour au moins un autre paramètre et est conçue pour calculer un signal de risque correspondant à une probabilité d'un grave dysfonctionnement cardiaque sur la base d'une combinaison linéaire des paramètres, comme celle qui est déterminée de préférence par la régression logistique, ou par combinaison non linéaire des paramètres.